(19) Europäisches Patentamt
European Patent Office
Office européen des brevets

(11) Veröffentlichungsnummer: **0 276 767 B1**

(12) **EUROPÄISCHE PATENTSCHRIFT**

(45) Veröffentlichungstag der Patentschrift: **08.04.92**

(51) Int. Cl.[5]: **C07C 45/65**, C07C 45/67, C07C 49/04, C07C 49/29, C07C 49/76

(21) Anmeldenummer: **88100888.2**

(22) Anmeldetag: **22.01.88**

# (54) Verfahren zur Herstellung von Ketonen.

(30) Priorität: **28.01.87 DE 3702483**

(43) Veröffentlichungstag der Anmeldung:
**03.08.88 Patentblatt 88/31**

(45) Bekanntmachung des Hinweises auf die Patenterteilung:
**08.04.92 Patentblatt 92/15**

(84) Benannte Vertragsstaaten:
**BE CH DE FR GB LI NL**

(56) Entgegenhaltungen:
**GB-A- 745 946**
**GB-A- 762 421**

**CHEMICAL ABSTRACTS, Band 105, Nr. 1, Juli 1986, Seite 579, Zusammenfassung Nr. 6184w, Columbus, Ohio, US; & JP-A-61 05 038**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Hupfer, Leopold, Dr.**
**Waltershoehe 3**
**W-6701 Friedelsheim(DE)**
Erfinder: **Schneider, Kurt, Dr.**
**Auf dem Koeppel**
**W-6702 Bad Duerkheim(DE)**

EP 0 276 767 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Herstellung von Ketonen durch Dehydratisierung und gleichzeitige Hydrierung in Gegenwart von Zeolithen des Pentasiltyps.

Ketone sind wegen ihrer vielseitigen Verwendungsmöglichkeiten zur Herstellung verschiedenster Produkte gesuchte chemische Verbindungen.

Insbesondere bei der technischen Herstellung von asymmetrisch substituierten Ketonen ist man in der Regel auf die Kondensation unterschiedlicher organischer Säuren unter Decarboxilierung angewiesen, wie sie z.B. in DE-OS 27 58 113 beschrieben ist. Bei diesem Verfahren ist der Anfall von symmetrisch substituierten Ketonen und von Kohlendioxid von Nachteil.

Auch besteht die Möglichkeit, ungesättigte Ketone nach bekannten Methoden zu hydrieren. So ist z.B. bekannt, alpha, beta-ungesättigte Ketone durch Aldolkondensation von aliphatischen Ketonen und Formaldehyd in Gegenwart von Oxalsäure bzw. in Gegenwart von Kationenaustauschern und $H_2O$ unter erhöhtem Druck [GB-PS 993 389) oder durch Reaktion von Enolacetaten mit Ketonen in Gegenwart von Lewis-Säuren, wie $BF_3$, $TiCl_4$ oder durch Oxidation verzweigter Olefine in 50%iger Essigsäure und in Gegenwart von $PdCl_2$ als Katalysator [BE-PS 658 801) oder durch Kondensation von gesättigten Carbonylverbindungen in Gegenwart von $BF_3$ herzustellen. Daneben gibt es ein mehrstufiges Verfahren, bei dem man alpha, beta-ungesättigte Ketone durch Kondensation von Ketonen in Gegenwart von Tosylmethylisocyanid, anschließende Alkylierung und Hydrolyse erhält. Andere aufwendige Herstellverfahren sind z.B. die Ozonolyse von 2,3-Dimethylbutadiene oder die anodische Oxidation von beta-Ketocarboxilaten oder die Photoisomerisaten von 1,2-Diacylcylobutanen. Die Gasphasenoxidation von Olefinen an Metalloxid-Phosphorixid-Katalysatoren führt unselektiv zu einem Gemisch verschiedener Verbindungen, unter denen sich auch alpha, beta-ungesättigte Ketone befinden. Diese Verfahren zur Herstellung der ungesättigten Ketone weisen verschiedene Nachteile auf, sei es, daß man von schwer zugänglichen Ausgangsverbindungen ausgehen, daß man toxische und korrosive Homogenkatalysatoren verwenden, oder daß man eine energieaufwendige oder mehrstufige Reaktionsführung, wie Ozonolyse bzw. Photoisomerisation in Kauf nehmen muß.

Ein Nachteil der Herstellung von gesättigten Ketonen aus Vorläufern der ungesättigten Ketonen ist die zweistufige Verfahrensweise, wobei in erster Stufe die ungesättigten Ketone und in der zweiten Stufe deren Hydrierung erfolgt.

Daraus ergab sich die Aufgabe, gesättigte Ketone, insbesondere asymmetrisch substituierte Ketone durch eine einfache Reaktion aus gut zugänglichen Ausgangsverbindungen zu synthetisieren.

Es wurde nun gefunden, daß man insbesondere asymmetrisch substituierte Ketone der Formel (I)

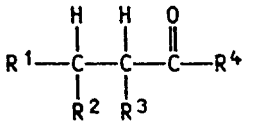

(I)

in der $R^1$ bis $R^4$ Wasserstoff und $R^1$ bis $R^4$ Alkyl- mit 1 bis 12 Kohlenstoffatomen, Cycloalkyl- mit 4 -8 Kohlenstoffatomen, Aryl-, Aralkyl- bzw. Alkylreste, die ihrerseits substituiert sein können, bedeuten bzw. $R^1$ und $R^2$ oder $R^2$ und $R^3$ zusammen mit den C-Atomen, an die sie gebunden sind, ein Cycloalkan bilden können, durch Dehydratisierung und Hydrierung in einem einzigen Verfahrensschritt bei hohen Selektivitäten, Umsätzen und Standzeiten erhält man, wenn man Ketone der Formel II

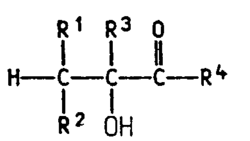

(II)

in der $R^1$ bis $R^4$ obige Bedeutung haben, mit Wasserstoff in Gegenwart von Zeolithen des Pentasiltyps, die zumindest eine Hydrierkomponente tragen, umsetzt.

Ketone der Formel (II), die erfindungsgemäß als Ausgangsstoffe umgesetzt werden, sind z.B. 3-Methyl-3-hydroxi-butan-2-on, 3-Methyl-3-hydroxi-pentan-2-on, 3-Pentamethylen-3-hydroxi, propan-2-on sowie auch 3-Methyl-3-hydroxi-heptan-2-on und 3-Phenyl-3-hydroxi-3-methylpropan-2-on. Die Ausgangsstoffe kann man z.B. nach der in DE-PS 11 29 941 beschriebenen Verfahrensweise aus tert.-Acetylalkoholen herstellen.

So gewinnt man erfindungsgemäß beispielsweise aus 3-Methyl-3-hydroxi-butan-2-on unter Abspaltung von $H_2O$ und Zuführung von $H_2$ das Methylisopropylketon.

Als saure Katalysatoren mit Hydrierkomponente werden bei dem erfindungsgemäßen Verfahren vorzugsweise Zeolithe verwendet, zweckmäßig in der aciden Form. Zeolithe sind kristalline Aluminiumsilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium ganz oder teilweise auch andere Elemente, wie B, Ga, Fe, Cr, V, As, Sb, Bi oder Be oder deren Gemische in das Gitter eingebaut werden, oder das Silicium kann durch ein vierwertiges Element, wike Ge, Ti, Zr, Hf ganz oder teilweise ersetzt werden.

Entsprechend ihrer Struktur werden die Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe.

In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind mannigfach beschrieben.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen, wie 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck hergestellt. Hierzu gehören auch die isotaktischen Zeolithe nach EP 34727 und EP 46504. Die erhaltenen Aluminosilikatzeolithe enthalten je nach Wahl ein $SiO_2/Al_2O_3$- Verhältnis von 10 bis 40 000` Aluminosilikatzeolithe des Pentasiltyps können auch in etherischem Medium wie Diethylenglykoldimethylether, in alkoholischem Medium, wie Methanol bzw. 1,4-Butandiol oder in Wasser synthetisiert werden.

Zu den erfindungsgemäß verwendbaren siliciumreichen Zeolithen ($SiO_2/Al_2O_3 \geqq 10$) gehören auch die verschiedenen ZSM-Typen, Ferrierit, Nu-1 und Silicalit®.

Borosilikatzeolithe kann man z.B. bei 90 bis 200°C unter autogenem Druck synthetisieren, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetraminlösung mit und insbesondere ohne Alkali- oder Erdalkalizusatz zur Reaktion bringt. Hierzu gehören auch die isotaktischen Zeolithe nach EP 34727 und EP 46504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer geeigneten Eisenverbindung, vorzugsweise $Fe_2[SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200°C unter autogenem Druck.

Die so hergestellten Alumino-, Boro- und Eisensilikatzeolithe können nach ihrer Isolierung, Trocknung bei 108 bis 160°C und Calcinierung bei 450 bis 5500°C, vorzugsweise 500°C, mit einem Bindemittel im Verhältnis 90:10 bis 40 bis 60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis

von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110°C/16 h getrocknet und bei 500°C/16 h calciniert.

Geeignete Katalysatoren erhält man auch, wenn die isolierten Alumino- bzw. Borosilikatzeolithe direkt nach der Trocknung verformt und erst nach der Verformung einer Calcinierung unterworfen werden. Die hergestellten Alumino- und Borosilikatzeolithe können in reiner Form, ohne Binder, als Stränge oder Tabletten eingesetzt werden, wobei als Verstrangungs- oder Peptisierungshilfsmittel, z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann dieser durch Ionenaustausch, z.B. mit Ammoniumionen und anschließende Calcinierung oder durch Behandlung mit Säuren vollkommen oder partiell in die gewünschte H-Form überführt werden.

Als Hydrierkomponente werden auf den verformten oder unverformten Zeolithen Übergangsmetalle, wie Cr, Mo, W, Mn, Re, V, Nb, insbesondere Metalle der 8. Gruppe, wie Fe, Co, Ni, Ru, Rh, Pd, Os, Ir, Pt und der 1. und 2. Nebengruppe, wie Cu, Ag, Zn oder deren Gemische aufgebracht. Diese Dotierung kann durch Ionenaustausch oder Imprägnierung mit Metallsalzen erfolgen.

Zweckmäßigerweise führt man diese Dotierung so durch, daß man den verformten Zeolithen in einem Steigrohr vorlegt und bei 20 bis 100°C eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise einer abermalige Calcinierung an.

Eine mögliche Ausführungsform besteht z.B. darin, daß man $Cu(NO_3)_2$ x 3 $H_2O$ oder $Ni(NO_3)_2$ x 6 $H_2O$ oder $Ce(NO_3)_3$ x 6 $H_2O$ oder $La(NO_3)_2$ x 6 $H_2O$ oder $Pd(NO_3)_2$ in Wasser löst und mit dieser Lösung den verformten oder unverformten Zeolith eine gewisse Zeit, z.B. 30 Minuten, tränkt. Die eventuell überstehende Lösung wird im Rotationsverdampfer von Wasser befreit. Danach wird der getränkte Zeolith bei etwa 150°C getrocknet und bei etwa 550°C calciniert. Dieser Tränkvorgang kann mehrmals hintereinander vorgenommen werden, um den gewünschten Metallgehalt einzustellen.

Es ist auch möglich, eine wäßrige $Ni(NO_3)_2$-Lösung oder ammoniakalische $Pd(NO_3)_2$-Lösung herzustellen und darin den reinen pulverförmigen Zeolithen bei 40 bis 100°C unter Rühren etwa 24 h aufzuschlämmen. Nach Abfiltrieren, Trocknen bei etwa 150°C und Calcinierung bei etwa 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige $Ni[NO))_2$-Lösung oder ammoniakalische $Pd[NO_3)_2$-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert. Bei manchen metalldotierten Zeolithen, z.B. Pd-, Cu-, Ni-dotierten Zeolithen ist eine Nachbehandlung mit Wasserstoff vorteilhaft.

In manchen Fällen ist es auch vorteilhaft, den Katalysator vor Reaktionsbeginn zu reduzieren. Beispielsweise ein Pt-, Pd- oder Cu-dotierter Zeolith-Katalysator wird im Reaktor auf 170 bis 220°C unter Stickstoff aufgeheizt, dann wird langsam Wasserstoff zugefügt. Die Temperatur wird so lange konstant gehalten bis kein Wasser mehr entsteht. Die Reduktion kann auch nach der Vorschrift, wie sie in J. of Catal. 89, 520 - 526 (1984) beschrieben ist, vorgenommen werden.

Man kann auch durch Imprägnierung und Ionenaustausch gleichzeitig mehrere Metalle als Hydrierkomponenten aufbringen.

Um eine möglichst hohe Selektivität, hohe Umsätze, sowie lange Standzeiten zu erreichen, ist es vorteilhaft, die Zeolithe zu modifizieren. Eine geeignete Modifizierung der Katalysatoren besteht z.B. darin, daß man zusätzlich zu den oben genannten Hydrierkomponenten die unverformten oder verformten Zeolithe mit Metallsalzen durch einen Ionenaustausch oder durch Imprägnierung dotiert. Als Metalle werden Alkalimetalle, wie Li, Cs, K, Erdalkalimetalle, wie Mg, Ca, Sr, Ba, Metalle der 3., 4, und 5. Hauptgruppe, wie B, Al, Ga, Ge, Sn, Pb, Bi, seltene Erdmetalle wie La, Ce, Pr, Nd, Er, Yb und U eingesetzt. Die Modifizierung mit diesen Metallen kann gleichzeitig mit der Aufbringung der Hydrierkomponente durch Ionenaustausch oder Imprägnierung erfolgen oder sich an die Aufbringung der Hydrierkomponente und eventuell an eine nachfolgende Calcination anschließen.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. An diese Art der Modifizierung schließt sich dann die Aufbringung der Hydrierkomponenten an.

Im einzelnen geht man vorteilhaft, z.B. so vor, daß man die Zeolithe in Pulverform mit 1 n Phosphorsäure 1 Stunde bei 80°C behandelt. Nach dieser Behandlung wird mit Wasser gewaschen, bei 110°C/16 Stunden getrocknet und bei 500°C/20 Stunden calciniert. Nach einer anderen Arbeitsweise behandelt man die Zeolithe vor oder nach ihrer Verformung mit Bindemittel, z.B. 1 bis 3 Stunden bei Temperaturen von 60 bis 80°C mit einer 3 bis 25 Gew.%igen, insbesondere 12 bis 20 Gew.%igen wäßrigen Salzsäure. Anschließend wird der so behandelte Zeolith mit Wasser gewaschen, getrocknet und bei 400°C bis 500°C calciniert. Nunmehr erfolgt die Aufbringung der Hydrierkomponente durch Ionenaustausch bzw. Imprägnierung wie zuvor beschrieben.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von 0,5 bis 5, vorzugsweise 1 bis 3 Stunden. Nach Isolierung, z.B. durch Abfiltrieren und Auswaschen des zeolithischen Materials, wird dieses zweckmäßig bei Temperaturen von 100 bis 160°C, getrocknet und bei Temperaturen von 450°C bis 600°C calciniert. Man kann die Säurebehandlung des zeolithischen Materials aber auch nach einer Verformung mit Bindemittel bei erhöhter Temperatur, zweckmäßig bei Temperaturen von 50 bis 90°C, vorzugsweise 60 bis 80°C über einen Zeitraum von 0,5 bis 5 h, mit 12 bis 20 Gew.%iger Salzsäure, vornehmen. Zweckmäßig wird das zeolithische Material anschließend ausgewaschen, bei Temperaturen von 100 bis 160°C getrocknet und bei Temperaturen von 450 bis 600°C calciniert. An eine HF-Behandlung kann auch eine HCl-Behandlung angeschlossen werden. Danach erfolgt die Aufbringung der Hydrierkomponente wie voranstehend beschrieben.

Nach einer anderen Arbeitsweise kann man Zeolithe durch Aufbringen von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110°C getrocknet und bei 500°C calciniert. Hieran schließt sich die Aufbringung der Hydrierkomponente an.

Wenn bei der erfindungsgemäßen Verwendung der zeolithischen Katalysatoren, wie auch bei den anderen gleichzeitig eingesetzten Katalysatoren eine durch Koksabscheidung bedingte Desaktivierung eintritt, empfiehlt es sich, diese durch Abbrennen der Koksablagerung mit Luft oder mit einem Luft/Stickstoff-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Die hier beschriebenen Katalysatoren können wahlweise als 2- bis 4mm-Stränge oder als Tabletten mit 3 bis 5 mm Durchmesser oder als Splitt mit Teilchengrößen von 0,1 bis 0,5 mm oder als Wirbelkontakt eingesetzt werden.

Die für die erfindungsgemäße Umwandlung der Hydroxyketone, in der Regel gewählten Reaktionsbedingungen sind in der bevorzugten Gasphase 100 bis 500°C, z.B. 150 bis 450°C und insbesondere 300 bis ,400°C und eine Katalysatorbelastung WHSV von 0,1 bis 20 $h^{-1}$, insbesondere von 1,0 bis $10,0^{-1}$ g Ausgangsstoff je g Katalysator und Stunde.

Das Verhältnis Wasserstoff zu Keton ist 1:1 bis 100:1 Mol, insbesondere 3:1 bis 30:1 Mol.

Die Gasphasenreaktion kann in einem Festbett oder in einem Wirbelbett ausgeführt werden.

Es ist auch möglich, die Reaktion in der Flüssigphase, in Suspension-, Riesel- oder Sumpffahrweise, bei Temperaturen zwischen 50 und 200°C durchzuführen.

Das Verfahren wird im allgemeinen bei Normaldruck oder je nach Flüchtigkeit der Ausgangsverbindung bei vermindertem oder mäßig erhöhtem Druck diskontinuierlich, vorzugsweise kontinuierlich durchgeführt.

Schwerflüchtige oder feste Ausgangsstoffe kann man in gelöster Form, z.B. in THF-, Toluol- oder Petrolether-Lösung einsetzen. Auch eine Verdünnung des Ausgangsstoffes mit diesen Lösungsmitteln oder mit Inertgasen, wie $N_2$, Ar, $H_2O$-Dampf ist möglich.

Nach der Umsetzung werden die entstandenen Produkte durch übliche Verfahren, z.B. durch Destillation aus dem Reaktionsgemisch isoliert; nicht umgesetzte Ausgangsstoffe werden gegebenenfalls in die Umsetzung zurückgeführt.

Beispiele 1 bis 10

Die Reaktion wird in der Gasphase unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsstoffe erfolgt gaschromatographisch.

Für die Beispiele werden folgende Katalysatoren eingesetzt.

Katalysator A

Ein Borosilikatzeolith des Pentasil-Typs wird in einer hydrothermalen Synthese aus 640 g hochdispersem $SiO_2$, 122 g $H_3BO_3$, 8 000 g einer wäßrigen 1,6-Hexandiamin-Lösung (Mischung 50:50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$. Daraus werden durch Verformen mit einem Verformungshilfsmittel 2 mm-Stränge hergestellt, die bei 110 °C/16 h getrocknet und bei 500 °C/24 h calciniert werden.

Katalysator A erhält man, indem man diese Stränge mit einer wäßrigen $Cu(NO_3)_2$-Lösung imprägniert, danach bei 130 °C/2 h trocknet und bei 540 °C/2 h calciniert. Der Cu-Gehalt beträgt 3,4 Gew.%.

Katalysator B

Katalysator B wird wie Katalysator A hergestellt, jedoch mit wäßriger $Pd(NO_3)_2$-Lösung statt $Cu(NO_3)_2$-Lösung getränkt. Der Pd-Gehalt beträgt 1,4 Gew.%.

Katalysator C

Die Stränge des Borosilikatzeolithen, wie bei Katalysator A beschrieben, werden in einer Kolonnne vorgelegt und einem Ionenaustausch mit einer ammoniakalischen Palladiumnitrat-Lösung bei 50 °C unterworfen. Nach Auswaschen mit $H_2O$ wird bei 110 °C getrocknet und bei 500 °C/5 h calciniert. Der Pd-Gehalt beträgt 1,9 Gew.%.

Katalysator D

Katalysator D wird wie Katalysator A hergestellt, jedoch mit einer wäßrigen Lösung aus Pd- und Ce-Nitrat anstatt Cu-Nitrat getränkt. Der Pd-Gehalt bedträgt 0,47 Gew.%, der Ce-Gehalt 2,3 Gew.%.

Katalysator E

Katalysator E wird wie Katalysator D hergestellt. Der Pd-Gehalt beträgt 1,3 Gew.%, der Ce-Gehalt 3,6 Gew.%.

Katalysator F

Katalysator F wird wie Katalysator D hergestellt, jedoch mit einer wäßrigen Lösung aus Pd- und Pr-Nitrat getränkt. Der Pd-Gehalt beträgt 1 Gew.%, der $PrO_2$-Gehalt 4,6 Gew.%.

Katalysator G

$Al_2O_3$ (D 10-11®, BASF) wird mit einer Pd-Nitratlösung getränkt. Der Pd-Gehalt beträgt 0,47 Gew.%.

Katalysator H

Katalysator H besteht aus einem $Al_2O_3$/MgO-Träger, der mit einer Pd-Nitratlösung imprägniert wurde. Der Katalysator H enthalt 1,12 Gew.% Pd, 19,8 Gew.% MgO und 78,1 Gew.% $Al_2O_3$.

Katalysator I

Katalysator I wird wie Katalysator F hergestellt, jedoch wird als Trägermaterial $Al_2O_3$ [D 10-11®, BASF) eingesetzt. Der Pd-Gehalt beträgt 0,5 Gew.%, der $PrO_2$-Gehalt 5,0 Gew.%.

Tabelle 1

| Beispiel | 1 | 2 | 3 | 4 | 5 | 6 | 7 | 8 | 9 | 10 |
|---|---|---|---|---|---|---|---|---|---|---|
| Katalysator | A | A | B | C | D | E | F | G | H | I |
| Temperatur | 350°C | 400°C | 375°C | 350°C | 375°C | 375°C | 375°C | 375°C | 375°C | 375°C |
| WHSV | 2,5h-1 | 2,5h-1 | 2h-1 | 2h-1 | 2h-1 | 2h-1 | 2h-1 | 2h-1 | 2h-1 | 2,5h-1 |
| $H_2$:Eduktmolar * | 8:1 | 8:1 | 15:1 | 10:1 | 10:1 | 10:1 | 10:1 | 10:1 | 10:1 | 8:1 |
| Umsatz % | 98,1 | 100 | 99,5 | 100 | 100 | 100 | 100 | 100 | 71,1 | 97,8 |
| Selektivität | | | | | | | | | | |
| Methylisopropylketon | 97,1 | 96,4 | 75,8 | 98,9 | 94,9 | 98,6 | 82,2 | 46,9 | 62,7 | 5,5 |
| Methylisopropenylketon | 1,4 | 0,4 | 22,5 | 0,5 | 0,3 | / | 9,4 | 31,8 | 14,9 | 76,2 |

Aus den Beispielen Tabelle erkennt man, daß unter den Katalysatoren für das erfindungsgemäße Verfahren die Zeolithe besonders gut geeignet sind.

* 3-Methyl-3-hydroxi-butan-2-on.

## Patentansprüche

**1.** Verfahren zur Herstellung von Ketonen der Formel (1)

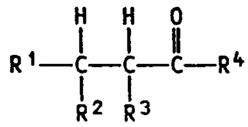 (I)

in der $R^1$ bis $R^3$ Wasserstoff und $R^1$ bis $R^4$ Alkyl- mit 1 bis 12 Kohlenstoffatomen, Cycloalkyl- mit 4 - 8 Kohlenstoffatomen, Aryl-, Aralkyl- bzw. Alkylarylreste, die ihrerseits substituiert sein können, bedeuten bzw. $R^1$ und $R^2$ und $R^3$ zusammen mit den C-Atomen, an die sie gebunden sind, ein Cycloalkan bilden können, dadurch gekennzeichnet, daß man Ketone der Formel (III)

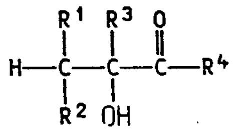 (II)

in der die Reste $R^1$ bis $R^4$ obige Bedeutung haben, mit Wasserstoff in Gegenwart von Zeolithen des Pentasiltyps, die zumindest eine Hydrierkomponente tragen, umsetzt.

2. Verfahren nach Anspruch 1 , dadurch gekennzeichnet, daß man als Hydrierkomponente ein Übergangsmetall und/oder Edelmetall oder deren Gemische verwendet.

3. Verfahren nach den Ansprüchen 1 und 2, dadurch gekennzeichnet, daß man Katalysatoren verwendet, die zusätzlich zu den Hydrierkomponenten mit Alkalimetallen, Erdalkalimetallen oder mit seltenen Erden dotiert sind.

4. Verfahren nach den Ansprüchen 1 bis 3, dadurch gekennzeichnet, daß man die Umsetzung in der Gasphase ausführt.

**Claims**

1. A process for preparing a ketone of formula (I)

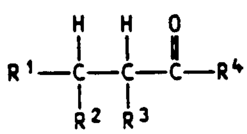 (I)

where R' to $R^3$ are each hydrogen and $R^1$ to $R^4$ are each alkyl of 1 to 12 carbon atoms, cycloalkyl of 4 to 8 carbon atoms, aryl, aralkyl or alkylaryl, which each in turn may be substituted, or $R^1$ and $R^2$ or $R^2$ and $R^3$ together with the carbon atoms to which they are bonded can form a cycloalkane, which comprises reacting a ketone of the formula (II)

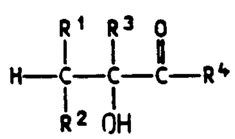 (II)

where $R^1$ to $R^4$ are each as defined above, with hydrogen in the presence of a zeolite of the pentasil type supporting one or more hydrogenation components.

2. A process as claimed in claim 1, wherein the hydrogenation component used is a transition metal or a noble metal or a mixture thereof.

3. A process as claimed in claim 1 or 2, wherein the catalyst used has in addition to the hydrogenation component been doped with an alkali metal, an alkaline earth metal or a rare earth.

4. A process as claimed in any of claims 1 to 3, wherein the reaction is carried out in the gas phase.

**Revendications**

1. Procédé de préparation de cétones de formule (1)

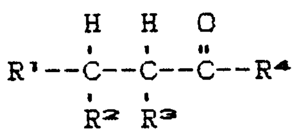

(I)

dans laquelle $R^1$ à $R^3$ peuvent représenter des atomes d'hydrogène et $R^1$ à $R^4$ peuvent représenter des restes alkyle à 1-12 atomes de carbone, cycloalkyle à 4-8 atomes de carbone, aryle, aralkyle ou alkylaryle, qui peuvent être substitués à leur tour, ou bien $R^1$, $R^2$ et $R^3$ peuvent former ensemble, avec les atomes de carbone auxquels ils sont fixés, un reste cycloalcane, caractérisé en ce qu'on fait réagir des cétones de formule (II)

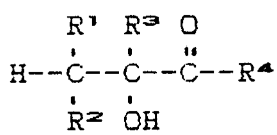

(II)

dans laquelle les restes $R^1$ à $R^4$ ont les significations données ci-dessus, avec de l'hydrogène en présence de zéolithes du type pentasil qui portent au moins un composant d'hydrogénation.

2. Procédé selon la revendication 1, caractérisé en ce qu'on utilise, comme composant d'hydrogénation, un métal de transition et/ou un métal précieux ou des mélanges de ces métaux.

3. Procédé selon la revendication 1 ou 2, caractérisé en ce qu'on utilise des catalyseurs qui, en plus des composants d'hydrogénation, sont dopés avec des métaux alcalins, des métaux alcalino-terreux ou avec des terres rares.

4. Procédé selon l'une quelconque des revendications 1 à 3, caractérisé en ce qu'on conduit la réaction en phase gazeuse.